# EUROPÄISCHE PATENTANMELDUNG

(11) **EP 4 640 814 A1**
(43) Veröffentlichungstag der Anmeldung: **29.10.2025**
(21) Anmeldenummer: 24171897.2
(22) Anmeldetag: 23.04.2024
(51) Int. Cl.: C12M 1/107, C12M 1/12, C12M 1/00, C12M 1/34, C12M 1/36

(54) **VORRICHTUNG UND VERFAHREN ZUR STEIGERUNG DER UMSATZRATEN BEI DER BIOLOGISCHEN METHANISIERUNG**

(71) Anmelder: Prüf- und Forschungsinstitut Pirmasens e.V., 66953 Pirmasens (DE)
(72) Erfinder: PACAN, Benjamin, 66957 Trulben (DE); SCHADEWELL, Christian, 66978 Merzalben (DE)
(74) Vertreter: Patentanwälte Dr. Keller, Schwertfeger Partnerschaft mbB

(57) **Zusammenfassung**

Die vorliegende Erfindung betrifft eine Vorrichtung und Verfahren zur Steigerung der Umsatzraten bei der biologischen Methanisierung für die Erzeugung von Methan aus Wasserstoff und CO₂-haltigem Gas. Die Vorrichtung umfasst eine Kolonne (10) mit im Inneren des Kolonnenkörper angeordneten strukturierten Packungen (12) für den Stoffaustausch zwischen unterschiedlichen Phasen, einen Auslass für erzeugtes methanhaltige Produktgas (14) und einen Einlass für flüssiges Nährmedium (16) im Kopfbereich der Kolonne und einen Auslass für das flüssige Nährmedium (26) und einen Einlass für Wasserstoff (24) im Fußbereich der Kolonne.

Unterhalb der strukturierten Packungen (12) ist ein Flüssigkeitsraum (20) für das flüssige Nährmedium vorgesehen, dessen Flüssigkeitsspiegel (32) im Flüssigkeitsraum (20) im Betriebszustand über das Befüllungsvolumen variabel regelbar ist. Im oberen Bereich des Flüssigkeitsraums (20) ist wenigstens eine schräg nach unten in den Flüssigkeitsraum (20) verlaufende Belüftungslanze (22) vorgesehen, über die das CO₂-Gas in den Flüssigkeitsraum (20) der Kolonne (10) beaufschlagbar ist, während eine Beaufschlagung von Wasserstoff in den Flüssigkeitsraum (20) über den darunter beabstandeten Einlass für Wasserstoff (24) erfolgt. Mit der Vorrichtung und dem Verfahren ist es möglich, die in die Flüssigkeit beaufschlagte CO₂-Menge und deren pH-Wert über eine Änderung des Flüssigkeitsspiegels im Flüssigkeitsraum zu regulieren.

## Beschreibung

Die vorliegende Erfindung betrifft eine Vorrichtung und ein Verfahren zur Steigerung der Umsatzraten bei der biologischen Methanisierung für die Erzeugung von Methangas aus Wasserstoff und CO₂-Gas.

Die biologische Methanisierung beruht auf der Aktivität methanogener Mikroorganismen (Archaeen), die Kohlenstoffdioxid (CO₂) und Wasserstoff (H₂) zu Methan (CH₄) und Wasser (H₂O) umwandeln. Allgemein wird als Methanisierung die Reaktion nach dem Sabatier-Prozess gemäß der Reaktionsgleichung 4H₂ + CO₂ → CH₄ + 2H₂O bezeichnet. Dieser unter Sauerstoffausschluss ablaufende, exotherme Prozess wird auch als Methanogenese bezeichnet. Üblicherweise wird der Prozess in einem Methanisierungsreaktor umgesetzt. Durch die Einstellung günstiger Betriebsund Milieubedingungen innerhalb des Methanisierungsreaktors werden hohe Umsatzraten an erzeugtem Methan erreicht. Besonders effizient sind leicht saure bis alkalische pH-Werte von 6.8 - 8.5 und eine Temperatur von 55° bis 70°C. Die Umsetzung von Wasserstoff und Kohlenstoffdioxid zu Methan und Wasser erfolgt über methanogene Bakterien. Für den bakteriellen Stoffwechsel werden neben ATP als Energiequelle Kohlenstoff und Nährstoffe wie Stickstoff, Schwefel und Phosphor benötigt.

In solchen Methanisierungsreaktoren werden Wasserstoff und Kohlenstoffdioxid unter Druck kontinuierlich zugeführt. Der Wasserstoff kann beispielsweise aus einer Elektrolyse stammen. Kohlenstoffdioxid kann beispielsweise in Form von Biogas zugeführt werden, wo es in einem größeren Anteil enthalten ist. Bei bisherigen Methanisierungsreaktoren wurde das Verhältnis der zugegebenen Menge von Wasserstoff zu Kohlenstoffdioxid kontinuierlich geregelt, sodass das Kohlenstoffdioxid nach Umsetzung im Tank nahezu verbraucht ist. Die bei der Umwandlung entstehende Wärme des exothermen Prozesses kann beispielsweise zur Gewinnung von Energie genutzt werden.

Bei bekannten Anlagen wird versucht, die gesamte Menge an Kohlenstoffdioxid mit Wasserstoff zu Methan umzusetzen, damit im Anschluss an die Methanisierungsreaktion keine Gastrennung notwendig ist, um die geforderten Qualitätskriterien, beispielsweise zur Einspeisung in ein Erdgasnetz, zu erfüllen. Hohe Umsatzraten sind jedoch nur mit einem Überschuss an Wasserstoff möglich. Sofern es zu einer Einspeisung des hergestellten Methans in das Gasnetz kommen soll, muss der überschüssige Wasserstoff abgetrennt werden. Bekannte Methanisierungsanlagen sind häufig nicht in der Lage, flexibel auf die tages- und jahreszeitlich unterschiedlich anfallenden Mengen an Wasserstoff aus Überschussstrom zu reagieren.

Die WO2023/212754A1 beschreibt beispielsweise ein Verfahren zur Herstellung von Methan aus einem kohlenstoffdioxidhaltigen Gasgemisch, mit dem es möglich ist, das in Biomasse gebundene Kohlenstoffdioxid möglichst vollständig unter Verwendung von Wasserstoff nutzbar zu machen. Die Vorrichtung umfasst einen Methanisierungsreaktor, in dem Kohlenstoffdioxid mit Wasserstoff durch anaerobe Fermentation umgesetzt wird. Über eine Zuleitung wird ein kohlenstoffdioxidhaltiger und ein wasserstoffhaltiger Edukt-Gasstrom in den Methanisierungsreaktor eingebracht. Über eine Ableitung wird der durch die anaerobe Fermentation erhaltene methanhaltige Produktgas-Strom aus dem Methanisierungsreaktor abgeleitet. Eine Gastrenneinrichtung trennt den Produktgas-Strom in eine wasserstofffreie, methanreiche Fraktion, sowie eine kohlenstoffdioxidreiche Fraktion auf. Eine Rückführeinrichtung führt die Kohlenstoffdioxidreiche Fraktion zum Edukt-Gasstrom zurück. Die Steigerung des molaren Verhältnisses des in dem Methanisierungsreaktor eingebrachten Wasserstoffes zum eingebrachten Kohlenstoffdioxid wird über eine Steuerungseinrichtung vorgenommen, die ein vorbestimmtes Verhältnis der beiden Gase regulieren kann. Abhängig von der verfügbaren Menge an Wasserstoff kann die Vorrichtung in Volllast oder Teillast betrieben werden. Zur Erzielung hoher Umsatzraten bei der Erzeugung von Methan ist diese Vorrichtung jedoch nicht geeignet, da diese bei Wasserstoffüberschuss zurückregeln würde.

Um möglichst hohe Umsatzraten zu erzielen, das heißt hohe Methanausbeuten, ist es erforderlich, das stöchiometrische Verhältnis zwischen Kohlenstoffdioxid und Wasserstoff stets aufrechtzuerhalten, sodass aus vier Wasserstoffmolekülen und einem Molekül Kohlenstoffdioxid ein Methanmolekül entstehen kann. In Gegenstromreaktoren ist dies jedoch problematisch, da aufgrund der im Vergleich zu Wasserstoff besseren Löslichkeit von Kohlenstoffdioxid in Flüssigkeit schon bei einem kleine Partialdruck Kohlensäure (H₂CO₃) entsteht, was zu einer Absenkung des pH-Wertes in der Flüssigkeit führt. Der niedrige pH führt letztendlich zu einer Versauerung des Mediums und macht den Prozess der Methanisierung uneffektiv.

Von diesem Hintergrund ist es Aufgabe der vorliegenden Erfindung, eine Vorrichtung und ein Verfahren bereitzustellen, mit der/dem es möglich ist, die Umsatzraten bei der biologischen Methanisierung zur Erzeugung von Methan aus Wasserstoff und Kohlenstoffdioxid auf einen Wasserstoffüberschuss zu maximieren, ohne dass es zu einer Übersäuerung des pH-Wertes im Flüssigkeitsmedium kommt.

Diese Aufgabe wird gelöst durch eine erfindungsgemäße Vorrichtung und ein Verfahren, indem die Menge an gelösten Kohlenstoffdioxid in dem flüssigen Nährmedium für die methanogenen Mikroorganismen über den Flüssigkeitsspiegel in der Kolonne des Methanisierungsreaktors reguliert wird.

Die erfindungsgemäße Vorrichtung zur Steigerung der Umsatzraten bei der biologischen Methanisierung für die Erzeugung von Methan aus Wasserstoff und CO₂-Gas, umfasst
- eine Kolonne mit im Inneren des Kolonnenkörper angeordneten strukturierten Packungen für den Stoffaustausch zwischen unterschiedlichen Phasen,
- einen Auslass für erzeugtes methanhaltige Produktgas und einen Einlass für flüssiges Nährmedium im Kopfbereich der Kolonne,
- einen Auslass für das flüssige Nährmedium und wenigstens einen Einlass für Wasserstoff im Fußbereich der Kolonne.

Die Vorrichtung ist dadurch gekennzeichnet, dass unterhalb der strukturierten Packungen ein Flüssigkeitsraum für das flüssige Nährmedium vorgesehen ist, dessen Flüssigkeitsspiegel im Flüssigkeitsraum im Betriebszustand über das Befüllungsvolumen variabel regelbar ist. Im oberen Bereich des Flüssigkeitsraums ist wenigstens eine, vorzugsweise mehrere, schräg nach unten in den Flüssigkeitsraum verlaufende Belüftungslanze vorgesehen, über die das CO₂-Gas in den Flüssigkeitsraum der Kolonne beaufschlagbar ist, während eine Beaufschlagung von Wasserstoff in den Flüssigkeitsraum über den darunter beabstandeten Einlass für Wasserstoff erfolgt.

Ziel des erfindungsgemäßen Verfahrens ist es, gleichbleibend hohe Umsatzraten, bevorzugt Maximalausbeuten, bei der Methanproduktion zu erzielen. In anderen Worten: aus einer Volumeneinheit Kohlenstoffdioxid pro Zeiteinheit soll dieselbe Volumeneinheit Methan pro Zeiteinheit erzeugt werden. Beispielweise ermöglichen es die erfindungsgemäße Vorrichtung und das Verfahren, dass aus 100 m³ CO₂ pro Stunde Eduktgas eine Menge von 100 m³ CH₄ pro Stunde Produktgas erzeugt werden. Erfindungsgemäß wird dies durch einen Wasserstoffüberschuss erreicht, d.h. es wird ein Vielfaches der stöchiometrischen Menge von Wasserstoff in Flüssigkeit gelöst. Vorzugsweise beträgt der Wasserstoffüberschuss mindestens das 4-fache, vorzugsweise 6-fache, mehr bevorzugt 8-fache der stöchiometrisch benötigten Menge gemäß der Summenformel 4H₂ + CO₂ → CH₄ + 2H₂O. Ziel ist es, denn Wasserpartialdruck sehr hoch zu halten.

Der hier verwendete Ausdruck CO₂-Gas umfasst sowohl ein kohlenstoffdioxidhaltiges Gasgemisch, als auch reines Kohlenstoffdioxid (CO₂). Beispiel für ein kohlenstoffdioxidhaltiges Gasgemisch sind Schwachgase, Klärgase oder Gase aus Biogasanlagen. Vorzugsweise wird bei der erfindungsgemäßen Vorrichtung und dem Verfahren ein CO₂-Gas in Form eines Gasgemisches verwendet, das einen CO₂-Gehalt von wenigstens 80% aufweist. Ein solches CO₂haltiges Gas wird auch als Schwachgas bezeichnet. In einer alternativen Ausführungsvariante wird als CO₂-Gas reines CO₂ verwendet.

Der hier verwendete Ausdruck Produktgas umfasst Methangas oder eine Methangasmischung, die normalerweise neben Methan auch noch Anteile an Kohlenstoffdioxid und Wasserstoff enthält. Bei dem Produktgas handelt es sich vorzugsweise um ein methanhaltiges Gasgemisch, vorzugsweise mit einem Methangehalt von > 80 %.

Erfindungsgemäß wird die Umsatzrate zur Erzeugung von Methangas bei der biologischen Methanisierung dadurch gesteigert, dass der Eintrag von CO₂-Gas in dem Methanisierungsreaktor in die Nährflüssigkeit erfolgt, wobei der Spiegel der Flüssigkeit, d.h. das Flüssigkeitsniveau, in dem Flüssigkeitsraum variabel regulierbar ist. Die Höhe des Flüssigkeitsspiegels ist im Betriebszustand des Reaktors daher durch Abnahme oder Zunahme des Flüssigkeitsvolumens in der Flüssigkeitskammer bei Bedarf absenkbar oder anhebbar. Über den variablen Flüssigkeitsspiegel lässt sich nun die Eintauchtiefe von Begasungseinrichtungen, d.h. Begasungslanzen, steuern, mit denen das flüssige Nährmedium mit Kohlenstoffdioxid beaufschlagt wird. Erfindungsgemäß wird die Eintauchtiefe der Begasungslanzen verringert, d.h. der Flüssigkeitsspiegel in der Kammer sinkt, wenn der pH-Wert unterhalb eines zuvor festgelegten Grenzwertes fällt und das Medium zu sauer wird. Ziel dieser Maßnahme ist es, den pH-Wert in dem flüssigen Nährmedium zu regulieren, um dadurch eine Übersäuerung des flüssigen Nährmediums zu verhindern, was zu einer geringeren Produktivität bei der Methanerzeugung führen würde.

Für den Stoffaustausch an den Phasen zwischen den von unten nach oben strömenden H₂- und CO₂-Gasströmen und den methanogenen Bakterien umfasst die erfindungsgemäße Vorrichtung eine Kolonne mit im Inneren des Kolonnenkörpers angeordneten, strukturierten Packungen. Ziel ist es, die an den strukturierten Packungen besiedelten Bakterien ausreichend mit Wasserstoff zu versorgen, während auf der anderen Seite ein Überschuss an CO₂ verhindert werden soll, da zu hohe CO₂-Konzentrationen zu einem hohen pH-Wert führen würden, welche das Wachstum der Bakterien beeinträchtigen und damit die Methanausbeute vermindern würden.

Strukturierte Packungen besitzen im Vergleich zu losen Füllkörpern einige Vorteile. Zum einen weisen die miteinander verschweißten Packungen eine höhere Druckfestigkeit auf, was größere Packungshöhen erlaubt, ohne dass diese durch Zwischenböden unterbrochen werden müssen. Dadurch sind in Kolonnen Packungshöhen von bis zu zehn Metern möglich. Da die strukturierten Packungen in einer Ausführungsvariante aus miteinander zu Paketen verschweißten, profilgeprägten Kunststofffolien bestehen, erfolgt eine gleichmäßigere Flüssigkeitsverteilung in der Kolonne. Durch diese Prägung und die Struktur, die mithilfe der Neigung der Folien im Innenbereich der Packungen entsteht, ist eine gleichmäßigere Verteilung der Flüssigkeit gewährleistet. Das gilt sowohl für niedrige, als auch sehr hohe Flüssigkeitsbeladungen. Ein weiterer Vorteil ist, dass Gas und Flüssigkeit aufgrund des wellenförmigen Profils während des Durchlaufes ständig miteinander vermischt werden, sodass die Grenzfläche zwischen Gas und Flüssigkeit ständig erneuert und hohe Stoffaustauschraten erzielt werden. Neben einem geringeren Druckverlust erlauben strukturierte Packungen somit höhere Durchsatzkapazitäten im Vergleich zu regellosen Schüttungen. Zudem können strukturierte Packungen in Kreuz-, Gitteroder Vertikalstruktur angeordnet werden, und somit speziell auf die Einsatzbedingungen abgestimmt werden. In bevorzugten Ausführungsformen können strukturierte Blechpackungen zum Einsatz kommen, beispielsweise Anordnungen gewellter Bleche, die eine Kreuzkanalstruktur bilden. In alternativen Varianten kommen Mellapak-Segmente zum Einsatz. Strukturierte Kunststoffpackungen bieten einen niedrigen Druckverlust und hohe Kapazitäten unter Prozessbedingungen, die vorzugsweise zu Einsatz kommen sollen. Der Vorteil dieser heiß gespritzten Packungen liegt in der Temperaturbeständigkeit, die diese Produktlinie von thermogeformten strukturierten Packungen unterscheidet.

Der Stoffaustausch in der Kolonne erfolgt im Gegenstromprinzip, das heißt, das in der Kolonne nach oben strömende Eduktgas führt zu einem Stoffaustausch am Phasenübergang mit der in der Kolonne herunterströmenden Flüssigkeit. Die Vorrichtung umfasst ferner einen Auslass für das erzeugte Methangas (Produktgas) und einen Einlass für das Nährmedium im Kopfbereich (d.h. im oberen Bereich) der Kolonne. Der Auslass für das Nährmedium und der Einlass für Wasserstoff befindet sich im Fußbereich (d.h. im unteren Bereich) der Kolonne. Die erfindungsgemäße Vorrichtung zeichnet sich dadurch aus, dass unterhalb der strukturierten Packungen ein flüssiges Nährmedium vorgesehen ist, dessen Flüssigkeitsspiegel bei Befüllung über das Befüllungsvolumen variabel regelbar ist. Vorzugsweise handelt sich bei dem Nährmedium um Nährflüssigkeit, in der die für die Methanisierung erforderlichen Nährstoffe enthalten sind. Um den Flüssigkeitsspiegel des Nährmediums im Flüssigkeitsraum zu regulieren, ist im oberen Bereich des Flüssigkeitsraumes wenigstens eine schräg nach unten in dem Flüssigkeitsraum verlaufende Belüftungslanze vorgesehen, über die das CO₂-haltige Gas in den Flüssigkeitsraum der Kolonne beaufschlagbar ist. Die Beaufschlagung des Gases über die Belüftungslanzen erfolgt zumindest teilweise in die Flüssigkeit, wobei die Menge des in der Flüssigkeit beaufschlagten CO₂-Gases abhängig ist von der Eintauchtiefe der wenigstens einen Belüftungslanze. Vorzugsweise umfasst die Belüftungslanze in deren Längserstreckung an der Oberfläche eine Vielzahl von Öffnungen für den Gasaustritt. Je tiefer die Belüftungslanze in die Flüssigkeit des Flüssigkeitsraumes eintaucht, umso mehr CO₂-haltiges Gas kann aus den Öffnungen direkt in die Flüssigkeit strömen und sich in der Flüssigkeit lösen. Gleichzeitig wird unterhalb der wenigstens einen Belüftungslanze Wasserstoff über einen Einlass in die Flüssigkeit des Flüssigkeitsraums beaufschlagt, d.h. in die Flüssigkeit eingeleitet, vorzugsweise über Belüfter oder Begasungslanzen. Wasserstoff ist hierbei schwerer in der Flüssigkeit löslich als Kohlenstoffdioxid. Vorzugsweise erfolgen der CO₂-Gas- und der H₂-Eintrag in die Flüssigkeit kontinuierlich, vorzugsweise mit einem gleichbleibenden Gasstrom. Vorzugsweise erfolgt der Eintrag von Wasserstoff in einem gegenüber Kohlenstoffdioxid stöchiometrischen Überschuss, vorzugsweise in einem wenigstens 4-fachen Überschuss, vorzugsweise 4- bis 8-fachen Überschuss gegenüber dem CO₂-Gas-Eintrag. Für diesen CO₂ reduzierenden Vorgang wird eine große Menge an H₂ benötigt, weswegen der Wasserstoffpartialdruck hier sehr hoch sein muss. Liegt H₂ nicht in ausreichender Konzentration vor, kann das vorliegende CO₂ nicht komplett umgewandelt werden und die Methangasproduktion ist nicht vollständig.

Angestrebt wird ein Methangehalt im Produktgas von wenigstens 80%, was durch die erfindungsgemäßen Maßnahmen zur CO₂-Gas-Löslichkeit gewährleistet wird. Die schräg im Flüssigkeitsraum angeordneten Belüftungslanzen und der variable Flüssigkeitsspiegel lösen das Problem der Übersäuerung des Mediums durch die Bildung von Kohlensäure, bedingt durch das Lösen von CO₂ in dem flüssigen Nährmedium. Denn ein kontinuierlicher CO₂-Strom würde aufgrund der guten Löslichkeit von CO₂ in Wasser zu einer fortschreitenden Absenkung des pH-Wertes und damit Übersäuerung des Mediums führen. Bei einer Absenkung des pH-Wertes von < 6 sinkt die Effizienz der Methanisierung und damit die Umsatzraten bei der Erzeugung von Methan. Durch die Regulation der Eintauchtiefe der Belüftungslanzen in die Flüssigkeit erfolgt eine Regulation des pH-Wertes, vorzugsweise ohne dass zusätzliche Puffersysteme, wie beispielsweise Phosphat-Puffer notwendig sind. Die Höhe des Flüssigkeitsspiegels und damit die Eintauchtiefe der Belüftungslanze kann somit zu einer effizienten pH-Regulation verwendet werden, ohne den CO₂-Eintrag abzusenken oder zu unterbrechen. Sinkt der pH-Wert unterhalb eines Grenzwertes, muss der Flüssigkeitsspiegel sinken, wodurch die Eintauchtiefe der wenigstens einen Belüftungslanze verringert wird. Ziel ist es, einen stabilen pH-Wert von vorzugsweise 7.2 im Medium zu erreichen.

Bei den Begasungslanzen handelt es sich vorzugsweise um Begasungseinrichtungen mit Öffnungen zum Gasaustritt. Vorzugsweise sind bei der erfindungsgemäßen Vorrichtung vier Begasungslanzen symmetrisch und radial entlang des Umfangs der Kolonne angeordnet. Bei einer bevorzugten Ausführungsvariante handelt es sich bei den Begasungslanzen um Rohrbelüfter. Bei einer alternativen Ausführungsvariante handelt es sich bei den Begasungslanzen um Membranoder Tellerbelüfter. Vorzugsweise sind die Begasungslanzen aus Edelstahl gefertigt, jedoch können auch Lanzen aus Kunststoff oder anderen geeigneten Materialien zum Einsatz kommen. Edelstahllanzen ermöglichen höhere Drücke. In alternativen Ausführungsvarianten sind die Begasungslanzen mit Düsen ausgestattet. Vorzugsweise sind möglichst eine Vielzahl von Öffnungen an der Oberfläche der Begasungslanze ausgebildet, um so eine möglichst große Austrittsfläche für das Gas zu ermöglichen. Bei den Öffnungen kann es sich entweder um Perforationen oder um Lamellen handeln, wie sie beispielsweise beim Membranlüfter zum Einsatz kommen. Die Begasungslanzen sind fest mit der Kolonne verbunden und somit statisch. In einer Ausführungsvariante wäre es auch möglich, den Flüssigkeitsspiegel konstant zu lassen und die Begasungslanzen variabel auszuführen. Dies würde jedoch einen erheblich größeren konstruktiven Aufwand erfordern. Auch ist zu beachten, dass die Begasungslanzen unter beträchtlichen Druck sind, sodass es wesentlich einfacher ist, den Flüssigkeitsspiegel zu verändern, statt die Lanzen zu bewegen. Vorzugsweise sind mehrere Begasungslanzen, vorzugsweise vier Begasungslanzen, in einem Winkel zwischen 30° und 50° nach unten im Flüssigkeitsraum radial an dem Kolonnenkörper angeordnet.

Ein weiterer Aspekt der erfindungsgemäßen Vorrichtung und des erfindungsgemäßen Verfahrens ist es, eine Unterversorgung mit Wasserstoff zu vermeiden. Daher erfolgt ein kontinuierlicher Einlassstrom von Wasserstoffgas in die Flüssigkeit des Flüssigkeitsraumes, sowie ein kontinuierlicher CO₂-Gas-Strom über die Begasungslanzen. Der Teil der Begasungslanze, der in die Flüssigkeit eingetaucht ist, setzt Kohlenstoffdioxid in der Flüssigkeit frei, welches sich zu Kohlensäure löst. Der nicht eingetauchte Teil der Begasungslanze setzt das Gas in das Kolonneninnere frei, so dass es von unten nach oben über die strukturierten Packungen zum Kopfbereich strömen kann, wo es zum Stoffaustausch mit den Mikroorganismen in der gegenströmenden Flüssigkeit kommt.

Um die Kolonne vorzugsweise mit einem 4- bis 8-fachen Überschuss von Wasserstoff zu versorgen und möglichst große Mengen an Wasserstoff in dem flüssigen Nährmedium zu lösen, ist in einer bevorzugten Ausführungsvariante eine Tauchglocke vorgesehen, die unterhalb der wenigstens einen Begasungslanze und oberhalb der Einlässe für Wasserstoff angeordnet ist. Die Tauchglocke dient der Anreicherung von Wasserstoffgas, indem sich das nach oben strebende Gas unterhalb der Glocke ansammelt, sodass der Wasserstoff bedingt durch die längere Verweilzeit in die Flüssigkeit in Lösung gehen kann. Vorzugsweise ist die Tauchglocke deshalb bogenförmig ausgestaltet, damit darunter der Sammelraum für das Wasserstoffgas entstehen kann. In einer bevorzugten Ausführungsvariante ist die Tauchglocke perforiert ausgeführt, sodass der sich darunter aufstauende Wasserstoff nach oben in der Flüssigkeit perlt, um in dieser in Lösung zugehen. Die Tauchglocke umfasst vorzugsweise eine Vielzahl von Perforationen, die vorzugsweise über die Oberfläche der Tauchglocke verteilt sind. Die Durchmesser der Perforationen können gleich sein oder auch variieren. Ziel der Perforationen ist es, dass das Wasserstoffgas feinperlig in die Flüssigkeit austreten kann, während die Flüssigkeit in der Kolonne von oben nach unten strömt. Durch hochfeine Perforationen wird eine große Grenzfläche wischen dem feinperlig ausströmenden Wasserstoffgas und der Flüssigkeit erreicht. In einer Ausführungsvariante besteht die Tauchglocke aus Blech oder Kunststoff mit darin angeordneten Perforationen. In einer alternativen Ausführungsform ist die Tauchglocke als Membranbelüfter ausgeführt, bei dem das Wasserstoffgas über die Kanäle in die Flüssigkeit perlt.

Der Umfang der Tauchglocke ist vorzugsweise kleiner als der Innenumfang der Kolonne, sodass wasserstoffangereichertes Nährmedium an der Glocke vorbei nach unten in Richtung Nährmediumausgang im Flüssigkeitssumpf gezogen wird, um somit von oben die Kolonne mit bereits wasserstoffangereichertem Nährmedium beregnen zu können.

Um die strukturierten Packungen ausreichend mit Wasserstoff zu versorgen, kann es erforderlich sein, Wasserstoff in der Mitte der Kolonne nachzuspeisen. Dafür befindet sich vorzugsweise im mittleren Bereich der Kolonne ein Einlass für die Nachspeisung von Wasserstoff in die strukturierten Packungen. Auf diese Weise wird die Wasserstoffmenge konstant hoch gehalten.

In einer weiteren Ausführungsvariante kann es erforderlich sein, dass zwischen den strukturierten Packungen Sammler- und Verregnungseinheiten für den Rückstau des Flüssigkeitsstroms vorhanden sind. Bei sehr langen Kolonnen mit großen Packungshöhen können auch mehrere solcher Sammler- und Verregnungseinheiten vorgesehen sein. Die Sammler- und Verregnungseinheit sorgt für eine Neuverteilung der Flüssigkeit, um der Randgängigkeit der Flüssigkeit entgegenzuwirken. Der Strom der Flüssigkeit von dem Kopfbereich in den Fußbereich der Kolonne wird dadurch umgelenkt und es verbleibt mehr Zeit für den Stoffaustausch und Methanbildung.

Die Vorrichtung enthält für die Steuerung vorzugsweise Sensoren zur pH-Wertmessung. So sind Sensoren zur Messung des pH-Wertes in der Flüssigkeit des Flüssigkeitsraumes vorgesehen. In einer weiteren Variante sind zudem pH-Messsensoren im Bereich der strukturierten Packungen vorgesehen. Zusätzlich ist die Vorrichtung mit Sensoren zur Messung der CO₂-Konzentration im erzeugten Produktgas bestückt.

Die vorliegende Erfindung betrifft auch Verfahren zur Steigung der Umsatzraten bei der biologischen Methanisierung für die Erzeugung von Methan aus Wasserstoff und CO₂-Gas durch methanogene Mikroorganismen, bei dem flüssiges Nährmedium für die Mikroorganismen von oben in eine Kolonne über strukturierte Packungen zu einem Ausgang im Flüssigkeitssumpf der Kolonne geleitet wird, während vom Fußbereich der Kolonne in den Kolonnenkörper beaufschlagtes CO₂-haltiges Gas und Wasserstoff im Gegenstrom von unten nach oben strömt, sodass zwischen den Phasen ein Stoffaustausch erfolgt. Das Absaugen der Flüssigkeit im Flüssigkeitssumpf erfolgt vorzugsweise über eine Umwälzpumpe. Das durch methanogene Mikroorganismen erzeugte methanhaltige Produktgas wird im Kopfbereich der Kolonne ausgeleitet. Unterhalb der strukturierten Packungen ist im Fußbereich der Kolonne ein Flüssigkeitsraum für das flüssige Nährmedium vorgesehen, dessen Flüssigkeitsspiegel durch das Befüllungsvolumen des Flüssigkeitsraums variabel geregelt wird. Hierbei wird das flüssige Nährmedium über wenigstens eine schräg nach unten verlaufende Belüftungslanze mit CO₂-Gas beaufschlagt, und unterhalb der wenigstens einen Belüftungslanze Wasserstoff in das flüssige Nährmedium geleitet. Durch diese Verfahrensmaßnahme werden die Menge des gelösten CO₂-Gases und der pH-Wert in dem flüssigen Nährmedium geregelt.

Das erfindungsgemäße Verfahren sieht in einer bevorzugten Variante vor, dass die Menge des in die Flüssigkeit beaufschlagten CO₂-Gases und/oder der pH-Wert des flüssigen Nährmediums über die Eintauchtiefe der wenigstens einen Belüftungslanze in das flüssige Nährmedium mittels des variablen Flüssigkeitsspiegels geregelt wird.

Mit dem erfindungsgemäßen Verfahren ist es möglich, aus der eingesetzten Molekülmenge CO₂ die gleiche Menge an Methan zu erzeugen. So wird aus 1 m³ CO₂ die äquivalente Menge von 1 m³ Methan erzeugt. Über die Eintauchtiefe der einen oder mehreren Begasungslanzen ist es möglich, bei gleichbleibendem CO₂-Strom den pH-Wert zu steuern und bei Bedarf anzuheben. Der CO₂-Gas-Strom bleibt dabei konstant, sodass immer hohe Umsatzraten erreicht werden, damit Wasserstoff in Überschuss vorliegt und ein hoher Wasserstoffpartialdruck erreicht wird. Erfindungsgemäß ist vorgesehen, dass dazu die Menge des einströmenden CO₂-Gases in das flüssige Nährmedium des Flüssigkeitsraums konstant gehalten wird, während Wasserstoff im stöchiometrischen Überschuss zugeleitet wird.

Da Wasserstoff im Vergleich zu Kohlenstoffdioxid schwerer in Flüssigkeiten lösbar ist, wird bei dem erfindungsgemäßen Verfahren eine Maßnahme eingesetzt, damit das Wasserstoffgas möglichst lange in der Flüssigkeit verbleibt. Erfindungsgemäß wird dies durch eine Tauchglocke umgesetzt, die unterhalb der wenigstens einen Belüftungslanze angeordnet wird, um so das Wasserstoffgas in der Flüssigkeit unterhalb der Glocke anzusammeln und die Verweildauer von Wasserstoff in der Flüssigkeit zu erhöhen. Zusätzlich wird der Wasserstoffpartialdruck in der Flüssigkeit erhöht. Durch die höhere Verweildauer hat das im Vergleich zu Kohlenstoffdioxid eher schwer lösliche Wasserstoffgas mehr Zeit, sich in der Flüssigkeit zu lösen. Vorzugsweise wird die Verweildauer von Wasserstoff im flüssigen Nährmedium verlängert, indem Wasserstoff mittels eine mehrfach perforierten Tauchglocke angereichert wird, sodass der Wasserstoff feinperlig in die Flüssigkeit in Lösung gehen kann.

Bei Bedarf kann zusätzlich eine Beaufschlagung von Wasserstoff in die strukturierten Packungen in Form einer Nachspeisung erfolgen, um den Wasserstoffpartialdruck hoch zu halten. In einer bevorzugten Variante umfasst die Tauchglocke eine Vielzahl von Perforationen, die vorzugsweise hochfein ausgeführt sind, d.h. einen im Verhältnis zur Oberfläche der Tauchglocke sehr kleinen Durchmesser aufweisen.

Als CO₂-Gas kommt vorzugsweise ein Schwachgas zur Anwendung, das heißt ein CO₂-haltiges Gasgemisch mit einem hohen Anteil von CO₂, das auch Anteile von CH₄ und H₂ enthalten kann. Vorzugsweise beträgt der Anteil von CO₂ im Schwachgas wenigstens etwa 80%. Der Anteil von CO₂ im Schwachgas kann allerdings auch schwanken, sodass es mithilfe des variablen Flüssigkeitsspiegels möglich ist, dem entgegenzusteuern. Das methanhaltige Produktgas ist vorzugsweise mit einer Gasaufbereitungsanlage gekoppelt, um eine höhere Methanreinheit zu erzielen. Der bei dem erfindungsgemäßen Verfahren eingesetzte Wasserstoff stammt vorzugsweise aus einer Druckelektrolyse. Mit dem erfindungsgemäßen Verfahren ist es möglich, den Partialdruck von Wasserstoff im System oder im Gasraum hochzuhalten, um so eine effiziente Diffusion über die Grenzflächen aufrecht zu erhalten. Vorzugsweise erfolgt eine Messung des pH-Wertes in dem flüssigen Nährmedium und eine Bestimmung der CO₂-Konzentration in dem erzeugten methanhaltigen Produktgas. Zur pH-Wertstabilisierung kann bedarfsweise das Puffersystem des Mediums durch eine Änderung des Verhältnisses der Phosphat-Salze angepasst werden. Damit können die Schwankungsbreiten bei einem pH-Wert deutlich verringert werden, sodass die pH-Werte > 7 sind.

Die Erfindung wird in dem nachführenden Ausführungsbeispiel näher erläutert.

In der gezeigten Ausführungsvariante ist eine Vorrichtung zur biologischen Methanisierung in Form eines Methanisierungsreaktors gezeigt. Diese besteht aus einer Kolonne 10 mit im Inneren des Kolonnenkörpers angeordneten strukturierten Packungen 12 für den Stoffaustausch zwischen unterschiedlichen Phasen. Im Kopfbereich der Kolonne 10 befindet sich ein Einlass 14 für Nährmedien sowie ein Auslass 16 für das methanhaltige Produktgas. Im Fußbereich der Kolonne 10 befindet sich unterhalb der strukturierten Packungen 12 ein Flüssigkeitsraum 20 der mit flüssigem Nährmedium befüllt ist. Der Flüssigkeitsspiegel 32 des Nährmediums ist variabel einstellbar, wodurch das Befüllungsvolumen der Flüssigkeit in dem Flüssigkeitsraum 20 regulierbar ist.

Erfindungsgemäß ist die Vorrichtung mit vier schräg nach unten angeordneten Begasungslanzen 22 bestückt. Die Begasungslanzen 22 besitzen eine Vielzahl von Öffnungen für den Gastransport an der Oberfläche. Über die Begasungslanzen 22 wird im Betriebszustand Schwachgas, also CO₂-haltiges-Gas in die Flüssigkeit des Flüssigkeitsraums 20 beaufschlagt. Über die Eintauchtiefe der Begasungslanzen 22 in die Flüssigkeit des Flüssigkeitsraums 20 sind die Menge des lösbaren CO₂ und der pH-Wert in der Flüssigkeit regelbar. Die Zugabe von Wasserstoff erfolgt im Fußbereich der Kolonne 10 über Einlässe 24, die unterhalb der Begasungslanzen 22 angeordnet sind. Unterhalb der Einlässe 24 für Wasserstoff ist der Flüssigkeitssumpf 28 zu sehen, in dem sich die durch Gravitation von oben nach unten über die strukturierten Packungen 12 strömende Flüssigkeit sammelt. Am unteren Ende des Flüssigkeitssumpfes 28 befindet sich der Auslass 26 für das Nährmedium.

Bei sehr hohen Packungshöhen der strukturierten Packungen 12 kann es erforderlich sein, dass eine oder mehrere Sammler- und Verregnungseinheiten 34 zum Einsatz kommen. Dadurch wird der Nährmedienstrom gleichmäßig über den Kolonnenquerschnitt verteilt, denn während des Durchfließens der Flüssigkeit durch die Packung 12 strebt die Flüssigkeit zum Kolonnenrand, während das aufsteigende Gas einen Zentralstrom in der Mitte der Kolonne 10 ausbilden könnte. Die Sammler- und Verregnungseinheit 34 sorgt für eine Neuverteilung der Flüssigkeit, um der Randgängigkeit entgegenzuwirken. Das von oben über den Einlass 14 in die Kolonne 10 beaufschlagte Nährmedium fließt unter Einfluss der Schwerkraft innerhalb der Kolonne 10 von oben nach unten, während das von unten über den Einlass 24 einströmende Wasserstoffgas sowie das über die Begasungslanzen 22 einströmende CO₂-Gas von unten nach oben aufsteigt. Während des Passierens der gestauten Flüssigkeit findet der Stoffaustausch zwischen den Phasen statt. In den strukturierten Packungen 12 findet der Stoffaustausch kontinuierlich innerhalb der Füllkörperschüttung oder der strukturierten Anordnung von Blechen statt. Um den Wasserstoffpartialdruck und damit die Wasserstoffsättigung hoch zu halten, kann Wasserstoff über einen zusätzlichen Einlass 30 im mittleren Bereich der Kolonne 10 nachgespeist werden.

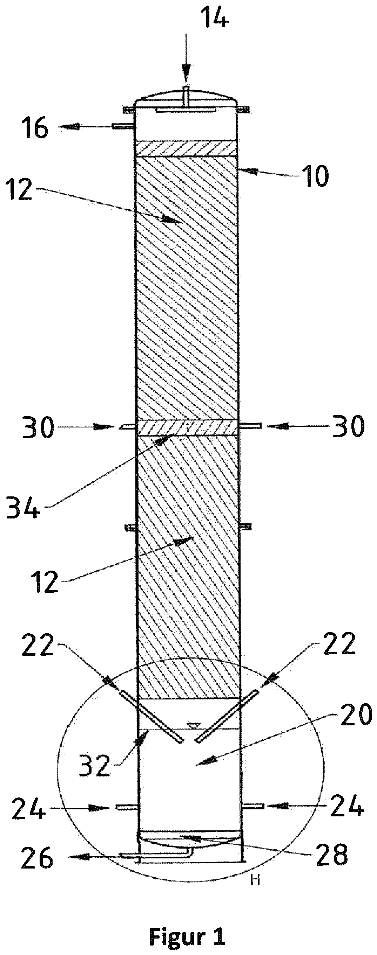

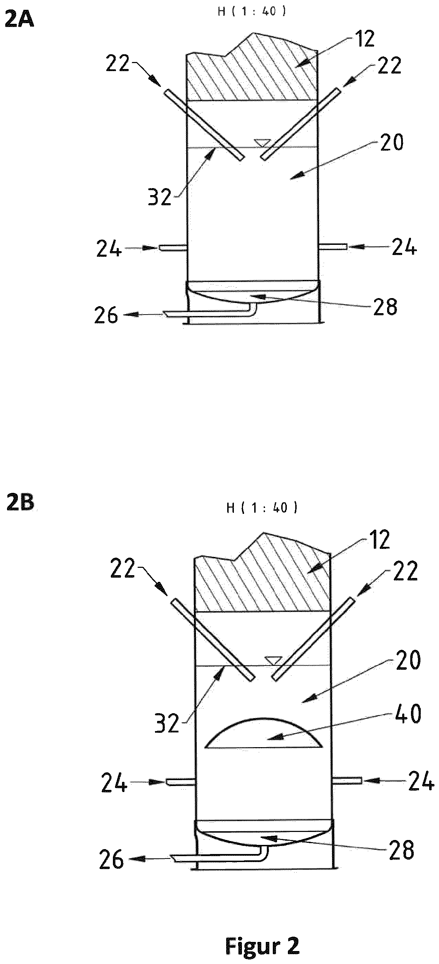

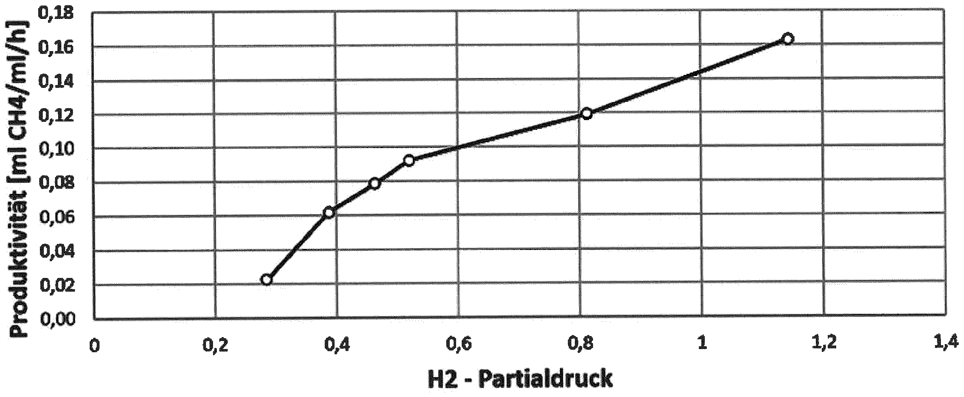

In Fig. 2A ist der Fußbereich der Kolonne 10 im Detail gezeigt. Deutlich sind die Begasungslanzen 22 zu sehen, die schräg in den Flüssigkeitsraum 20 eingebaut sind. Ferner sind die Einlässe 24 für Wasserstoff, der Flüssigkeitssumpf 28 sowie die Auslässe 26 für das Nährmedium zu erkennen. Der Flüssigkeitsraum 20 befindet sich unterhalb der strukturierten Packungen 12. Über den Auslass 26 für das Nährmedium kann der Flüssigkeitsspiegel 32 in dem Flüssigkeitsraum 20 kontrolliert und angepasst werden.

In Fig. 2B ist eine weitere bevorzugte Ausführungsvariante gezeigt. Hier ist zusätzlich in dem Flüssigkeitsraum 20 eine hochfein perforierte Tauchglocke 40 angeordnet, die sich zwischen den Enden der Begasungslanzen 22 und oberhalb der Einlässe 24 für Wasserstoff befindet. Die Tauchglocke 40 ist bogenförmig oder als Haube ausgestaltet und dient dazu, dass über die darunterliegenden Einlässe 24 für Wasserstoff einströmende Wasserstoffgas unterhalb der Tauchglocke 40 zu sammeln, um so eine längere Verweildauer des Wasserstoffgases in der Flüssigkeit und damit eine höhere Löslichkeit von Wasserstoff in der Flüssigkeit zu erzielen. Durch die feine Perforierung in der Tauchglocke 40 perlt der Wasserstoff nach oben in der Flüssigkeit, während wasserstoffangereichertes Nährmedium an der Glocke vorbei nach unten in Richtung Auslässe 26 gezogen wird, um somit von oben die Kolonne mit bereits wasserstoffangereichertem Nährmedium beregnen zu können.

Mithilfe der erfindungsgemäßen Vorrichtung und des Verfahrens ist es möglich, die Umsatzraten bei der biologischen Methanisierung zur Erzeugung von Methan aus Wasserstoff und CO₂-Gas deutlich zu steigern und die Methanisierung deutlich effizienter zu gestalten. Nahezu die gesamte dem System zugeführte Menge von CO₂ kann zu CH₄ umgesetzt werden, was bedingt ist durch den provozierten Wasserstoffüberschuss und einer gleichzeitigen Kontrolle des pH-Wertes mittels Anpassung des Flüssigkeitsspiegels 32 in dem Flüssigkeitsraum 20 der Kolonne 10. Damit wird eine optimale Prozessführung möglich, wodurch konstant niedrige CO₂-Werte und hohe Wasserstoffpartialdrücke aufrechterhalten werden können.

In Fig. 3 ist deutlich zu sehen, wie sich die Produktivität mit einem höheren Wasserstoffpartialdruck steigert. Zumindest in dem getesteten Druckbereich zwischen 0.3 bis 1.2 Partialdruck erkennt man einen nahezu linearen Zusammenhang zwischen Partialdruck und Produktivität. Deutlich ist zu erkennen, wie eine Erhöhung des Partialdruckes von 0.5 auf 1.15 zu einer Verdoppelung der Produktivität führt. Die hohen Wasserstoffpartialdrücke wirken sich positiv auf das Redoxpotential, die Produktivität und über einen indirekten Einfluss auch auf den pH-Wert aus.

Durch eine Entkoppelung der Wasserstoffbegasung und Kohlenstoffdioxidbegasung ist somit eine deutliche Steigerung der Umsatzraten, das heißt der Produktivität möglich. Die erfindungsgemäße Ausgestaltung der CO₂-Zugabe in das Flüssigkeitsmedium durch Veränderung des Flüssigkeitsspiegels ermöglicht es, den CO₂-Gehalt im Prozess zu regeln. Durch die erfindungsgemäß eingesetzten Begasungslanzen wird die Wasserstoffzugabe im Fußbereich der Kolonne, das heißt im Kolonnenboden, durch die einhergehende Oberflächenvergrößerung und Geometrie der Begasungslanzen optimiert. Eine weitere Optimierung findet durch die perforierte Tauchglocke zur Wasserstoffrückhaltung statt. Die optionale Nachspeisung von Wasserstoff in die strukturierte Packung, beispielsweise im mittleren Bereich der Kolonne, führt zu einer zusätzlichen Optimierung des Prozesses und der Aufrechterhaltung eines hohen Wasserstoffpartialdruckes im System. Insgesamt ermöglicht es der erfindungsgemäße Aufbau und das Verfahren, dass eine gleichmäßige Versorgung der Mikroorganismen mit Wasserstoff und Kohlenstoffdioxid gewährleistet ist, sodass ein hoher Methangehalt im erzeugten Produktgas erreicht wird.

### Bezugszeichenliste

10 = Kolonne
12 = strukturierte Packungen
14 = Einlass für Nährmedium
16 = Auslass für methanhaltiges Produktgas
20 = Flüssigkeitsraum
22 = Begasungslanze
24 = Einlass für Wasserstoff
26 = Auslass für Nährmedium
28 = Flüssigkeitssumpf
30 = Einlass zur optionalen Nachspeisung von Wasserstoff
32 = variabler Flüssigkeitsspiegel
34 = Sammler- und Verregnungseinheit
40 = Tauchglocke zur Wasserstoffanreicherung

## Patentansprüche

1. Vorrichtung zur Steigerung der Umsatzraten bei der biologischen Methanisierung für die Erzeugung von Methan aus Wasserstoff und CO₂-Gas, umfassend
- eine Kolonne (10) mit im Inneren des Kolonnenkörper angeordneten strukturierten Packungen (12) für den Stoffaustausch zwischen unterschiedlichen Phasen,
- einen Auslass für erzeugtes methanhaltige Produktgas (14) und einen Einlass für flüssiges Nährmedium (16) im Kopfbereich der Kolonne,
- einen Auslass für das flüssige Nährmedium (26) und wenigstens einen Einlass für Wasserstoff (24) im Fußbereich der Kolonne,
**dadurch gekennzeichnet, dass** unterhalb der strukturierten Packungen (12) ein Flüssigkeitsraum (20) für das flüssige Nährmedium vorgesehen ist, dessen Flüssigkeitsspiegel (32) im Flüssigkeitsraum (20) im Betriebszustand über das Befüllungsvolumen variabel regelbar ist, wobei im oberen Bereich des Flüssigkeitsraums (20) wenigstens eine schräg nach unten in den Flüssigkeitsraum (20) verlaufende Belüftungslanze (22) vorgesehen ist, über die das CO₂-Gas in den Flüssigkeitsraum (20) der Kolonne (10) beaufschlagbar ist, während eine Beaufschlagung von Wasserstoff in den Flüssigkeitsraum (20) über den darunter beabstandeten Einlass für Wasserstoff (24) erfolgt.

2. Vorrichtung nach Anspruch 1, **dadurch gekennzeichnet, dass** am Umfang der wenigstens einen Belüftungslanze (22) eine Vielzahl von Öffnungen für den CO₂-Gastransport ausgebildet sind und dass mehrere Begasungslanzen (20) in einem Winkel zwischen 30° und 50° nach unten gerichtet in dem Flüssigkeitsraum (20) am Kolonnenkörper radial angeordnet sind.

3. Vorrichtung nach einem der Ansprüche 1 oder 2, **dadurch gekennzeichnet, dass** es sich bei den Begasungslanzen (22) um Rohrbelüfter oder Membranbelüfter oder Tellerbelüfter handelt.

4. Vorrichtung nach einem der Ansprüche 1 bis 3, **dadurch gekennzeichnet, dass** eine Regelungseinheit vorgesehen ist, mit der der Flüssigkeitsspiegel (32) des flüssigen Nährmediums in dem Flüssigkeitsraum (20) und damit die Eintauchtiefe der Begasungslanzen (22) in das flüssige Nährmedium in Abhängigkeit von der CO₂-Konzentration und/oder des pH-Wertes des flüssigen Nährmediums regelbar ist.

5. Vorrichtung nach einem der Ansprüche 1 bis 4, **dadurch gekennzeichnet, dass** unterhalb der wenigstens einen Begasungslanze (22) und oberhalb des Einlasses für Wasserstoff (24) eine Tauchglocke (40) zur Anreicherung von Wasserstoff vorgesehen ist, die vorzugsweise eine Vielzahl von Perforationen für den feinperligen Austritt von Wasserstoff in die Flüssigkeit aufweist.

6. Vorrichtung nach Anspruch 5, **dadurch gekennzeichnet, dass** die Tauchglocke (40) bogenförmig oder als Haube ausgestaltet ist und dass der Umfang der Tauchglocke (40) kleiner ist als der Innenumfang der Kolonne (10).

7. Vorrichtung nach einem der Ansprüche 1 bis 6, **dadurch gekennzeichnet, dass** im mittleren Bereich der Kolonne (10) ein zusätzlicher Einlass (30) für die Nachspeisung von Wasserstoff (30) in die strukturierten Packungen (12) angeordnet ist.

8. Vorrichtung nach einem der Ansprüche 1 bis 7, **dadurch gekennzeichnet, dass** zwischen den strukturierten Packungen (12) Sammler- und Verregnungseinheiten (34) zur Neuverteilung des Flüssigkeitsstroms in den nachfolgenden Packungen vorgesehen sind.

9. Verfahren zur Steigerung der Umsatzraten bei der biologischen Methanisierung für die Erzeugung von Methan aus Wasserstoff und CO₂-Gas durch methanogene Mikroorganismen, bei dem flüssiges Nährmedium für die Mikroorganismen von oben in eine Kolonne über strukturierte Packungen zu einem Ausgang im Flüssigkeitssumpf der Kolonne geleitet wird, während vom Fußbereich der Kolonne in den Kolonnenkörper beaufschlagtes CO₂-Gas und Wasserstoff im Gegenstrom von unten nach oben strömt, sodass zwischen den Phasen ein Stoffaustausch erfolgt, wobei das durch methanogene Mikroorganismen erzeugte methanhaltige Produktgas im Kopfbereich der Kolonne ausgeleitet wird, **dadurch gekennzeichnet, dass** unterhalb der strukturierten Packungen im Fußbereich der Kolonne ein Flüssigkeitsraum für das flüssige Nährmedium vorgesehen ist, dessen Flüssigkeitsspiegel durch das Befüllungsvolumen des Flüssigkeitsraums variabel geregelt wird, wobei das flüssige Nährmedium über wenigstens eine schräg nach unten verlaufende Belüftungslanze mit CO₂-Gas beaufschlagt wird, und unterhalb der wenigstens einen Belüftungslanze Wasserstoff in das flüssige Nährmedium geleitet wird, wodurch die Menge des gelösten CO₂-Gases und/oder der pH-Wert in dem flüssigen Nährmedium geregelt werden.

10. Verfahren nach Anspruch 9, **dadurch gekennzeichnet, dass** die Menge des in das flüssige Nährmedium beaufschlagten CO₂-Gases und/oder der pH-Wert des flüssigen Nährmediums über die Eintauchtiefe der wenigstens einen Belüftungslanze in das flüssige Nährmedium mittels des variablen Flüssigkeitsspiegels geregelt wird.

11. Verfahren nach einem der Ansprüche 9 oder 10, **dadurch gekennzeichnet, dass** die Verweildauer von Wasserstoff im flüssigen Nährmedium verlängert wird, indem Wasserstoff mittels einer mehrfach perforierten Tauchglocke angereichert wird, sodass der Wasserstoff feinperlig in die Flüssigkeit in Lösung gehen kann.

12. Verfahren nach einem der Ansprüche 9 bis 11, **dadurch gekennzeichnet, dass** die Menge des einströmenden CO₂-Gases in das flüssige Nährmedium des Flüssigkeitsraums konstant gehalten wird, während Wasserstoff im stöchiometrischen Überschuss zugeleitet wird.

13. Verfahren nach einem der Ansprüche 8 bis 12, **dadurch gekennzeichnet, dass** wenigstens eine Messung des pH-Wertes in dem flüssigen Nährmedium und eine Bestimmung der CO₂-Konzentration in dem erzeugten methanhaltigen Produktgas erfolgt.

14. Verfahren nach einem der Ansprüche 8 bis 13, **dadurch gekennzeichnet, dass** zusätzlich eine Beaufschlagung von Wasserstoff in die strukturierten Packungen erfolgt.
